# EUROPEAN PATENT SPECIFICATION

(11) **EP 3 240 894 B1**
(45) Date of publication and mention of the grant of the patent: **27.01.2021**
(21) Application number: 15874275.9
(22) Date of filing: 22.12.2015
(51) Int. Cl.: C12N 5/10, C12N 5/071, C12N 15/63

(54) **RECOMBINANT CELLS COMPRISING miRNA MIMICS**
REKOMBINANTE ZELLEN MIT miRNA- MIMETIKA
CELLULES RECOMBINANTES COMPRENANT DES MIMÉTIQUES DE miARN

(30) Priority: 23.12.2014 US 201462096016 P; 04.03.2015 US 201562127860 P
(43) Date of publication of application: 08.11.2017
(73) Proprietor: University of Georgia Research Foundation, Athens, GA 30602-7411 (US); Tripp, Ralph, A., Watkinsville, GA 30677 (US); Weilin, Wu, Watkinsville, GA 30677 (US)
(72) Inventor: TRIPP, Ralph, A., Watkinsville, GA 30677 (US); WEILIN, Wu, Watkinsville, GA 30677 (US)
(74) Representative: Moré, Solveig Helga
(86) International application number: PCT/US2015/067256
(87) International publication number: WO 2016/106268

(56) References cited:
- WO-A1-2014/159633
- WO-A2-2013/182553
- US-A1- 2012 137 379
- BARRON N ET AL: "Engineering CHO cell growth and recombinant protein productivity by overexpression of miR-7", JOURNAL OF BIOTECHNOLOGY, vol. 151, no. 2, 7 December 2010 (2010-12-07), pages 204-211, XP028171088, ISSN: 0168-1656, DOI: 10.1016/J.JBIOTEC.2010.12.005 [retrieved on 2010-12-15]
- WU D & MURASHOV A M: "MicroRNA-431 regulates axon regeneration in mature sensory neurons by targeting the Wnt antagonist Kremen1", FRONTIERS IN MOLECULAR NEUROSCIENCE, vol. 6, October 2013 (2013-10), XP055487680, ISSN: 1662-5099, DOI: 10.3389/fnmol.2013.00035
- KANAAN Z ET AL: "A plasma microRNA panel for detection of colorectal adenomas: a step toward more precise screening for colorectal cancer", ANNALS OF SURGERY, vol. 258, no. 3, September 2013 (2013-09), pages 400-408, XP009506394, ISSN: 1528-1140, DOI: 10.1097/SLA.0B013E3182A15BCC
- Anonymous: "microRNA (miRNA): Overview", , 25 June 2018 (2018-06-25), XP055487621, Retrieved from the Internet: URL:http://dharmacon.horizondiscovery.com/ rnai/microrna/#overview [retrieved on 2018-06-25]
- Anon.: "miRIDIAN(TM) microRNA Mimics, Hairpin Inhibitors and Negative Controls", , 21 January 2015 (2015-01-21), XP055487622, Retrieved from the Internet: URL:http://dharmacon.horizondiscovery.com/ uploadedFiles/Resources/miridian-mimics-in hibit-neg-contr-prodinsert.pdf [retrieved on 2018-06-25]
- JIN, J ET AL.: 'MICRORNA-501 PROMOTES HBV REPLICATION BY TARGETING HBXIP' BIOCHEMICAL AND BIOPHYSICAL RESEARCH COMMUNICATIONS vol. 430, no. 4, 25 December 2012, pages 1228 - 1233, XP055460680 DOI: 10.1016/J.BBRC.2012.12.071

## Description

### I. BACKGROUND

1. The demand of specific biotherapeutics, particularly monoclonal antibodies and therapeutic proteins and peptides, has been steadily increasing. This increased demand has partially been met through the use of high throughput of antibody generation utilizing phage display for in vitro selection which moved the major bottleneck to the production and purification of recombinant antibodies in an end-user friendly format. Nevertheless, such production and purification bottlenecks remain a significant hurdle for cost effective specific high affinity binding reagents. In particular, expression of mAb from mammalian cells has been shown to have low yield, medium complexity, and serum requirements. What are needed are new methods and compositions which remove the production and purification hurdles providing for reduced costs to the consumer.

### II. SUMMARY

2. Disclosed are methods and compositions related to recombinant cells comprising microRNA (miRNA) mimics, in particular, hsa-miR-431.

### III. BRIEF DESCRIPTION OF THE DRAWINGS

3. The accompanying drawings, which are incorporated in and constitute a part of this specification, illustrate several embodiments and together with the description illustrate the disclosed compositions and methods.

4. Figure 1 shows optimization of dsRNA transfection into CHO cells ∼90% transfection efficiency is achieved at a PPIB siRNA concentration of 20 nM in the presence of DF1 0.5%.

5. Figure 2 shows a miRNA mimic screen in CHO cells. CHO cells were reversed transfected with 20 nM of each miRNA mimic. 7 days post transfection, culture medium was collected and an ELISA was performed to assess the level of antibody production.

### IV. DETAILED DESCRIPTION

6. Before the present compounds, compositions, articles, devices, and/or methods are disclosed and described, it is to be understood that they are not limited to specific synthetic methods or specific recombinant biotechnology methods unless otherwise specified, or to particular reagents unless otherwise specified, as such may, of course, vary. It is also to be understood that the terminology used herein is for the purpose of describing particular embodiments only.

### A. Definitions

7. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

8. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10" as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

9. In this specification and in the claims which follow, reference will be made to a number of terms which shall be defined to have the following meanings:
10. "Optional" or "optionally" means that the subsequently described event or circumstance may or may not occur, and that the description includes instances where said event or circumstance occurs and instances where it does not.

### B. Compositions

12. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular microRNA mimic is disclosed and discussed and a number of modifications that can be made to a number of molecules including the microRNA mimic are discussed, specifically contemplated is each and every combination and permutation of microRNA mimic and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

13. Antibodies are host proteins that are produced by a terminally differentiated B-cell called a plasma cell in response to foreign molecules that enter the body. These foreign molecules are called antigens and their molecular recognition by the immune system results in selective production of antibodies that are able to bind the specific antigen. Antibodies generated in response to the antigen circulate throughout the blood and lymph where they bind to their specific antigen, enabling the antigen to be cleared from circulation through the activation of complement, natural killer cells, and macrophage, as well as, the presentation of antigenic peptides to effector T cells.

14. Antibodies have long been recognized for their therapeutic, prophylactic, diagnostic and research applications. However, difficulties with large scale production and purification of antibodies has been a significant barrier to their commercial use. The problems observed with respect to large scale production and purification of antibodies has also been observed in the production of other recombinant proteins and peptides, such as, for example, therapeutic enzymes. Disclosed herein are compositions and methods which overcome these barriers that hinder the commercial use of antibodies proteins, and peptides. Disclosed herein are recombinant cells comprising the microRNA (miRNA) mimic hsa-miR-431 and at least one nucleic acid encoding an immunoglobulin, exogenous protein, or exogenous peptide.

15. "microRNA" refers to short non-coding RNA molecules that regulate gene expression through a post-transcriptional mechanism. The molecules are transcribed as long precursor primary-miRNAs (pri-miRNA) that are then processed into complex hairpin structures (pre-miRNA) by the Drosha nuclease. Upon export from the nucleus, these hairpin structures are then cleaved to create short duplexes miRNAs by the cytoplasmic nuclease, Dicer. Typically, a microRNA will bind to a target mRNA in the 3' UTR of a coding region of a gene and can either inhibit or activate expression of the gene. It is understood and herein contemplated that the binding to the target does not have to be perfect and can occur where the microRNA recognizes as few as 2-7 complementary nucleotides. In one aspect, it is understood and herein contemplated that the disclosed hsa-miR-431 binds and supresses the expression of one or more host gene mRNAs that negatively regulate immunoglobulin production in the recombinant cell.

16. It is understood and herein contemplated that hsa-miR-431 alone or in combination with one two, three, four, five, or six other. microRNA mimics can be utilized in the recombinant cell. For example, the recombinant cell can comprise a single microRNA mimic hsa-miR-431, Similarly, the recombinant cell may comprise two, three, four, five, six, or seven different miRNA mimics. For example the recombinant cell can comprise hsa-miR-431 and hsa-miR-623; hsa-miR-431 and hsa-miR-4325; hsa-miR-431 and hsa-miR-3129; hsa-miR-431 and hsa-miR-3127; hsa-miR-431 and hsa-miR-873; hsa-miR-431 and hsa-miR-612 hsa-miR-431, hsa-miR-623, and hsa-miR-4325; hsa-miR-431, hsa-miR-623, and hsa-miR-3129; hsa-miR-431, hsa-miR-623, and hsa-miR-3127; hsa-miR-431, hsa-miR-623, and hsa-miR-873; hsa-miR-431, hsa-miR-623, and hsa-miR-612; hsa-miR-431, hsa-miR-4325, and hsa-miR-3129; hsa-miR-431, hsa-miR-4325, and hsa-miR-3127; hsa-miR-431, hsa-miR-4325, and hsa-miR-873; hsa-miR-431, hsa-miR-4325, and hsa-miR-612; hsa-miR-431, hsa-miR-3129, and hsa-miR-3127; hsa-miR-431, hsa-miR-3129, and hsa-miR-873; hsa-miR-431, hsa-miR-3129, and hsa-miR-612; hsa-miR-431, hsa-miR-3127, and hsa-miR-873; hsa-miR-431, hsa-miR-3127, and hsa-miR-612; hsa-miR-431, hsa-miR-873, and hsa-miR-612; hsa-miR-431, hsa-miR-623, hsa-miR-4325, and hsa-miR-3129; hsa-miR-431, hsa-miR-623, hsa-miR-4325, and hsa-miR-3127; hsa-miR-431, hsa-miR-623, hsa-miR-4325, and hsa-miR-873; hsa-miR-431, hsa-miR-623, hsa-miR-4325, and hsa-miR-612; hsa-miR-431, hsa-miR-623, hsa-miR-3129, and hsa-miR-3127; hsa-miR-431, hsa-miR-623, hsa-miR-3129, and hsa-miR-873; hsa-miR-431, hsa-miR-623, hsa-miR-3129, and hsa-miR-612; hsa-miR-431, hsa-miR-623, hsa-miR-3127, and hsa-miR-873; hsa-miR-431, hsa-miR-623, hsa-miR-3127, and hsa-miR-612; hsa-miR-431, hsa-miR-623, hsa-miR-873, and hsa-miR-612; hsa-miR-431, hsa-miR-4325, hsa-miR-3129, and hsa-miR-3127; hsa-miR-431, hsa-miR-4325, hsa-miR-3129, and hsa-miR-873; hsa-miR-431, hsa-miR-4325, hsa-miR-3129, and hsa-miR-612; hsa-miR-431, hsa-miR-4325, hsa-miR-3127, and hsa-miR-873; hsa-miR-431, hsa-miR-4325, hsa-miR-3127, and hsa-miR-612; hsa-miR-431, hsa-miR-4325, hsa-miR-873, and hsa-miR-612; hsa-miR-431, hsa-miR-3129, hsa-miR-3127, and hsa-miR-873; hsa-miR-431, hsa-miR-3129, hsa-miR-3127, and hsa-miR-612; hsa-miR-431, hsa-miR-3129, hsa-miR-873, and hsa-miR-612; hsa-miR-431, hsa-miR-3127, hsa-miR-873, and hsa-miR-612; hsa-miR-431, hsa-miR-623, hsa-miR-4325, hsa-miR-3129, and hsa-miR-3127; hsa-miR-431, hsa-miR-623, hsa-miR-4325, hsa-miR-3129, and hsa-miR-873; hsa-miR-431, hsa-miR-623, hsa-miR-4325, hsa-miR-3129, and hsa-miR-612; hsa-miR-431, hsa-miR-623, hsa-miR-4325, hsa-miR-3127, and hsa-miR-873; hsa-miR-431, hsa-miR-623, hsa-miR-4325, hsa-miR-3127, and hsa-miR-612; hsa-miR-431, hsa-miR-623, hsa-miR-4325, hsa-miR-873, and hsa-miR-612; hsa-miR-431, hsa-miR-623, hsa-miR-3129, hsa-miR-3127, and hsa-miR-873; hsa-miR-431, hsa-miR-623, hsa-miR-3129, hsa-miR-3127, and hsa-miR-612; hsa-miR-431, hsa-miR-623, hsa-miR-3129, hsa-miR-873, and hsa-miR-612; hsa-miR-431, hsa-miR-623, hsa-miR-3127, hsa-miR-873, and hsa-miR-612; hsa-miR-431, hsa-miR-4325, hsa-miR-3129, hsa-miR-3127, and hsa-miR-873; hsa-miR-431, hsa-miR-4325, hsa-miR-3129, hsa-miR-3127, and hsa-miR-612; hsa-miR-431, hsa-miR-4325, hsa-miR-3129, hsa-miR-873, and hsa-miR-612; hsa-miR-431, hsa-miR-4325, hsa-miR-3127, hsa-miR-873, and hsa-miR-612; hsa-miR-431, hsa-miR-3129, hsa-miR-3127, hsa-miR-873, and hsa-miR-612; hsa-miR-431, hsa-miR-623, hsa-miR-4325, hsa-miR-3129, hsa-miR-3127, and hsa-miR-873; hsa-miR-431, hsa-miR-623, hsa-miR-4325, hsa-miR-3129, hsa-miR-3127, and hsa-miR-612; hsa-miR-431, hsa-miR-623, hsa-miR-4325, hsa-miR-3129, hsa-miR-873, and hsa-miR-612; hsa-miR-431, hsa-miR-623, hsa-miR-4325, hsa-miR-3127, hsa-miR-873, and hsa-miR-612; hsa-miR-431, hsa-miR-623, hsa-miR-3129, hsa-miR-3127, hsa-miR-873, and hsa-miR-612; hsa-miR-431, hsa-miR-4325, hsa-miR-3129, hsa-miR-3127, hsa-miR-873, and hsa-miR-612; or hsa-miR-431, hsa-miR-623, hsa-miR-4325, hsa-miR-3129, hsa-miR-3127, hsa-miR-873, and hsa-miR-612. It is further understood and contemplated herein that there can be multiple copies of one or more of the miRNA mimics in the cell.

### 1. Nucleic Acids

Because the disclosed compositions comprise miRNA mimics, in a very broad sense, the compositions disclosed herein describe recombinant cells comprising nucleic acid molecules. It is understood and herein contemplated that there are a variety of molecules disclosed herein that are nucleic acid based as well as various functional nucleic acids. The disclosed nucleic acids are made up of for example, nucleotides, nucleotide analogs, or nucleotide substitutes. Non-limiting examples of these and other molecules are discussed herein. It is understood that for example, when a vector is expressed in a cell that the expressed mRNA will typically be made up of A, C, G, and U. Likewise, it is understood that if, for example, an antisense molecule is introduced into a cell or cell environment through for example exogenous delivery; it is advantageous that the antisense molecule be made up of nucleotide analogs that reduce the degradation of the antisense molecule in the cellular environment.

### a) Nucleotides and related molecules

17. A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenine-9-yl (A), cytosine-1-yl (C), guanine-9-yl (G), uracil-1-yl (U), and thymin-1-yl (T). The sugar moiety of a nucleotide is a ribose or a deoxyribose. The phosphate moiety of a nucleotide is pentavalent phosphate. A non-limiting example of a nucleotide would be 3'-AMP (3'-adenosine monophosphate) or 5'-GMP (5'-guanosine monophosphate).

18. As disclosed throughout this disclosure, in one aspect disclosed herein are recombinant cells comprising an hsa-miR-431 microRNA mimic. As used herein, a miRNA "mimic" is a synthetic nucleic acid molecule designed to interact with endogenous mRNAs or other nucleic acids. These mimics can comprise nucleic acid analogs, modifications, or comprise artificial double or triple nucleic acid strands. In general, the chemical and/or design modifications described herein are used to enhance the molecule's stability, deliverability, or specificity.

19. As used herein, a "nucleotide analog" is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl (.psi.), hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex formation. Often time base modifications can be combined with for example a sugar modification, such as 2'-O-methoxyethyl, to achieve unique properties such as increased duplex stability.

20. Nucleotide analogs can also include modifications of the sugar moiety. Modifications to the sugar moiety would include natural modifications of the ribose and deoxy ribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀, alkyl or C₂ to C₁₀ alkenyl and alkynyl. 2' sugar modiifcations also include but are not limited to -O[(CH₂)ₙO]ₘ CH₃, -O(CH₂)ₙ OCH₃, -O(CH₂)ₙ NH₂, -O(CH₂)ₙ CH₃, -O(CH₂)ₙ -ONH₂, and -O(CH₂)ₙON[(CH₂)ₙ CH₃)]₂, where n and m are from 1 to about 10.

21. Other modifications at the 2' position include but are not limted to: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications may also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs may also have sugar mimetics such as cyclobutyl moieties in place of the pentofuranosyl sugar.

22. Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkage between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included.

23. It is understood that nucleotide analogs need only contain a single modification, but may also contain multiple modifications within one of the moieties or between different moieties.

24. Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

25. Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones;formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts.

26. It is also understood in a nucleotide substitute that both the sugar and the phosphate moieties of the nucleotide can be replaced, by for example an amide type linkage (aminoethylglycine) (PNA).

27. It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethylammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety.

28. A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

29. A Hoogsteen interaction is the interaction that takes place on the Hoogsteen face of a nucleotide or nucleotide analog, which is exposed in the major groove of duplex DNA. The Hoogsteen face includes the N7 position and reactive groups (NH₂ or O) at the C6 position of purine nucleotides.

### b) Functional Nucleic Acids

30. In one respect, mimics are functional nucleic acids. Functional nucleic acids are nucleic acid molecules that have a specific function, such as binding a target molecule or catalyzing a specific reaction. Functional nucleic acid molecules can be divided into the following categories, which are not meant to be limiting. For example, functional nucleic acids include antisense molecules, aptamers, ribozymes, triplex forming molecules, and external guide sequences. The functional nucleic acid molecules can act as affectors, inhibitors, modulators, and stimulators of a specific activity possessed by a target molecule, or the functional nucleic acid molecules can possess a de novo activity independent of any other molecules. It is important to note that functional molecules in each category can adopt multiple designs. Thus, for instance, miRNA mimics can be single stranded, double stranded, can contain hairpin structures or contain multiple strands.

31. Functional nucleic acid molecules can interact with any macromolecule, such as DNA, RNA, polypeptides, or carbohydrate chains. Thus, functional nucleic acids can interact with mRNA or genomic DNA or they can interact with polypeptides. Often functional nucleic acids are designed to interact with other nucleic acids based on sequence homology between the target molecule and the functional nucleic acid molecule. In other situations, the specific recognition between the functional nucleic acid molecule and the target molecule is not based on sequence homology between the functional nucleic acid molecule and the target molecule, but rather is based on the formation of tertiary structure that allows specific recognition to take place.

32. Antisense molecules are designed to interact with a target nucleic acid molecule through either canonical or non-canonical base pairing. The interaction of the antisense molecule and the target molecule is designed to promote the destruction of the target molecule through, for example, RNAseH mediated RNA-DNA hybrid degradation. Alternatively the antisense molecule is designed to interrupt a processing function that normally would take place on the target molecule, such as transcription or replication. Antisense molecules can be designed based on the sequence of the target molecule. Numerous methods for optimization of antisense efficiency by finding the most accessible regions of the target molecule exist. Exemplary methods would be in vitro selection experiments and DNA modification studies using DMS and DEPC. It is preferred that antisense molecules bind the target molecule with a dissociation constant (k_{d}) less than or equal to 10⁻⁶, 10⁻⁸, 10⁻¹⁰, or 10⁻¹².

33. Aptamers are molecules that interact with a target molecule, preferably in a specific way. Typically aptamers are small nucleic acids ranging from 15-50 bases in length that fold into defined secondary and tertiary structures, such as stem-loops or G-quartets. Aptamers can bind small molecules, such as ATP and theophiline, as well as large molecules, such as reverse transcriptase and thrombin. Aptamers can bind very tightly with k_{d}s from the target molecule of less than 10⁻¹² M. It is preferred that the aptamers bind the target molecule with a k_{d} less than 10⁻⁶, 10⁻⁸, 10⁻¹⁰, or 10⁻¹². Aptamers can bind the target molecule with a very high degree of specificity. For example, aptamers have been isolated that have greater than a 10000 fold difference in binding affinities between the target molecule and another molecule that differ at only a single position on the molecule (United States patent 5,543,293). It is preferred that the aptamer have a k_{d} with the target molecule at least 10, 100, 1000, 10,000, or 100,000 fold lower than the k_{d} with a background binding molecule. It is preferred when doing the comparison for a polypeptide for example, that the background molecule be a different polypeptide.

34. Ribozymes are nucleic acid molecules that are capable of catalyzing a chemical reaction, either intramolecularly or intermolecularly. Ribozymes are thus catalytic nucleic acid. It is preferred that the ribozymes catalyze intermolecular reactions. There are a number of different types of ribozymes that catalyze nuclease or nucleic acid polymerase type reactions which are based on ribozymes found in natural systems, such as hammerhead ribozymes, hairpin ribozymes, and tetrahymena ribozymes. There are also a number of ribozymes that are not found in natural systems, but which have been engineered to catalyze specific reactions de novo (for example, but not limited to the following United States patents: 5,580,967, 5,688,670, 5,807,718, and 5,910,408). Preferred ribozymes cleave RNA or DNA substrates, and more preferably cleave RNA substrates. Ribozymes typically cleave nucleic acid substrates through recognition and binding of the target substrate with subsequent cleavage. This recognition is often based mostly on canonical or non-canonical base pair interactions. This property makes ribozymes particularly good candidates for target specific cleavage of nucleic acids because recognition of the target substrate is based on the target substrates sequence.

35. Triplex forming functional nucleic acid molecules are molecules that can interact with either double-stranded or single-stranded nucleic acid. When triplex molecules interact with a target region, a structure called a triplex is formed, in which there are three strands of DNA forming a complex dependent on both Watson-Crick and Hoogsteen base-pairing. Triplex molecules are preferred because they can bind target regions with high affinity and specificity. It is preferred that the triplex forming molecules bind the target molecule with a k_{d} less than 10⁻⁶, 10⁻⁸, 10⁻¹⁰, or 10⁻¹².

36. External guide sequences (EGSs) are molecules that bind a target nucleic acid molecule forming a complex, and this complex is recognized by RNase P, which cleaves the target molecule. EGSs can be designed to specifically target a RNA molecule of choice. RNAse P aids in processing transfer RNA (tRNA) within a cell. Bacterial RNAse P can be recruited to cleave virtually any RNA sequence by using an EGS that causes the target RNA:EGS complex to mimic the natural tRNA substrate. Similarly, eukaryotic EGS/RNAse P-directed cleavage of RNA can be utilized to cleave desired targets within eukarotic cells.

### 2. Antibodies

37. As noted throughout this disclosure, in addition to hsa-miR-431, the recombinant cells disclosed herein can comprise at least one immunoglobulin encoding nucleic acid. It is understood and herein contemplated that the disclosed immunoglobulin genes can encode for any immunoglobulin or immunoglobulin-like receptor including, but not limited to antibodies, antibody fragments, diabodies, bi-specific antibodies, variable lymphocyte receptors (VLRs), and antibody fusion constructs.

### (1) Antibodies Generally

38. The term "antibodies" is used herein in a broad sense and includes both polyclonal and monoclonal antibodies. In addition to intact immunoglobulin molecules, also included in the term "antibodies" are fragments or polymers of those immunoglobulin molecules, and human or humanized versions of immunoglobulin molecules or fragments thereof, as long as they are chosen for their ability to interact with a given target. The antibodies can be tested for their desired activity using the *in vitro* assays described herein, or by analogous methods, after which their *in vivo* therapeutic and/or prophylactic activities are tested according to known clinical testing methods. There are five major classes of human immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. One skilled in the art would recognize the comparable classes for mouse. The heavy chain constant domains that correspond to the different classes of immunoglobulins are called alpha, delta, epsilon, gamma, and mu, respectively. Thus, in one aspect, disclosed herein are recombinant cells comprising hsa-miR-431 and at least one immunoglobulin encoding nucleic acid, wherein the immunoglobulin encoded by the nucleic acid is an antibody.

39. The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity.

40. The disclosed monoclonal antibodies can be made using any procedure which produces mono clonal antibodies. For example, disclosed monoclonal antibodies can be prepared using hybridoma methods, such as those described by Kohler and Milstein. In a hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro.*

41. The monoclonal antibodies may also be made by recombinant DNA methods. DNA encoding the disclosed monoclonal antibodies can be readily isolated and sequenced using conventional procedures (e.g., by using oligonucleotide probes that are capable of binding specifically to genes encoding the heavy and light chains of murine antibodies). Libraries of antibodies or active antibody fragments can also be generated and screened using phage display techniques.

42. *In vitro* methods are also suitable for preparing monovalent antibodies. Digestion of antibodies to produce fragments thereof, particularly, Fab fragments, can be accomplished using routine techniques known in the art. For instance, digestion can be performed using papain. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

43. As used herein, the term "antibody or fragments thereof" encompasses chimeric antibodies and hybrid antibodies, with dual or multiple antigen or epitope specificities, and fragments, such as F(ab')2, Fab', Fab, Fv, sFv, scFv, and the like, including hybrid fragments. Thus, fragments of the antibodies that retain the ability to bind their specific antigens are provided. Such antibodies and fragments can be made by techniques known in the art and can be screened for specificity and activity according to the methods set forth in the Examples and in general methods for producing antibodies and screening antibodies for specificity and activity. Thus, in one aspect, disclosed herein are recombinant cells comprising hsa-miR-431 and at least one immunoglobulin encoding nucleic acid, wherein the immunoglobulin encoded by the nucleic acid comprises an antibody fragment such as F(ab')2, Fab', Fab, Fv, sFv, or scFv.

44. Also included within the meaning of "antibody or fragments thereof" are conjugates of antibody fragments and antigen binding proteins (single chain antibodies).

45. The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment..

46. In one embodiment, the encoded immunoglobulin may also refer to antibodies with multiple specificities such as bi or tri-specific antibodies. Such antibodies comprise a common Fc region, but are engineered so the variable regions are specific for different targets (bi-specific) and can be engineered to have an additional variable region (tri-specific). Accordingly, disclosed herein are recombinant cells comprising hsa-miR-431 and at least one immunoglobulin encoding nucleic acid, wherein the immunoglobulin encoded by the nucleic acid comprises a bi- or tri-specific antibody. hsa-miR-431.

47. As used herein, the term "antibody" or "antibodies" can also refer to a human antibody and/or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods serves to lessen the chance that an antibody administered to a human will evoke an undesirable immune response.

### (2) Human antibodies

48. The disclosed human antibodies can be prepared using any technique. The disclosed human antibodies can also be obtained from transgenic animals. Specifically, the homozygous deletion of the antibody heavy chain joining region (J*(H)*) gene in these chimeric and germ-line mutant mice results in complete inhibition of endogenous antibody production, and the successful transfer of the human germ-line antibody gene array into such germ-line mutant mice results in the production of human antibodies upon antigen challenge. Antibodies having the desired activity are selected using Env-CD4-co-receptor complexes as described herein.

### (3) Humanized antibodies

49. Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an sFv, Fv, Fab, Fab', F(ab')2, or other antigen-binding portion of an antibody) which contains a portion of an antigen binding site from a non-human (donor) antibody integrated into the framework of a human (recipient) antibody.

50. To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody.

51. Methods for humanizing non-human antibodies are well known in the art. For example, humanized antibodies can be generated according to the methods of Winter et al. by substituting rodent CDRs or CDR sequences for the corresponding sequences of a human antibody.

### (4) Immunoglobulin Fusion constructs

In one aspect, the disclosed immunoglobulin can be a fusion construct of an immunoglobulin variable region and another determinant such as a signal sequence or targeting moiety. In one aspect, disclosed herein are recombinant cells comprising hsa-miR-431 and at least one immunoglobulin encoding nucleic acid, wherein the immunoglobulin encoded by the nucleic acid comprises a immunoglobulin fusion construct.

### (5) Diabodies

52. As noted above, the disclosed nucleic acids may encode an immunoglobulin gene such as a diabody. As disclosed herein "diabody" refers to a single chain by specific immunoconstruct. Diabodies comprise two variable regions and no Fc region. The variable regions are specific for different targets. In one aspect, disclosed herein are recombinant cells comprising hsa-miR-431 and at least one immunoglobulin encoding nucleic acid, wherein the immunoglobulin encoded by the nucleic acid comprises a diabody.

### (6) Variable Lymphocyte Receptors

53. Variable lymphocyte receptors (VLRs) immunoglobulin-like molecules generated by jawless vertebrates (agnanthans) and are generated by RAG-independent combinatorial assembly of leucine-rich repeat cassettes for Ag recognition, instead of the Ig-based Ag receptors used by jawed vertebrates. The VLR genes encode for crescent-shaped proteins that use variable β-strands and a C-terminal loop to bind to Ags rather than the six CDR loops used by BCRs and TCRs. Each VLR transcript encodes an invariant signal peptide (SP) followed by highly variable LRR modules: a 27-34 residue N-terminal LRR (LRRNT), the first 24-residue LRR (LRR1), up to eight 24-residue variable LRRs (LRRV), one 24-residue end LRRV (LRRVe), one 16-residue connecting peptide LRR (LRRCP), and a 48-63 residue C-terminal LRR (LRRCT). In one aspect, disclosed herein are recombinant cells comprising hsa-miR-431 and at least one immunoglobulin encoding nucleic acid, wherein the immunoglobulin encoded by the nucleic acid comprises a VLR.

### 3. Recombinant Proteins and Peptides

54. Importantly, while the examples herein focus on antibody production, it is understood and herein contemplated that the production enhancements would extend to other exogenous proteins and peptides, including, but not limited to biotherapeutics such as biotherapeutic enzymes. For example, in one aspect, disclosed herein are recombinant cells comprising hsa-miR-431 and at least one nucleic acid encoding an exogenous protein or peptide, wherein the protein or peptide is a recombinant protein or peptide from the group consisting of Tissue Plasminogen Activator (TPA), Erythropoietin (EPO), Granulocyte colony-stimulating factor (G-CSF), Interferon, α-galactosidase A, α-L-iduronidase, N-acetylgalactosamine-4-sulfatase, Glucocerebrosidase, Dornase alfa, Hepatitis B virus Envelope (Env) Protein, Factor VII, recombinant human growth hormone (rHGH), biosynthetic human insulin (BSI), follicle-stimulating hormone (FSH), recombinant cholera toxin B, diphtheria toxoid, tetanus toxoid, pertussis toxoid, Hepatitis B surface antigen (HBsAg), HPV capsid protein, and recombinant Human Immunodeficiency Virus (HIV) proteins (Pol, gp41, gp120, gp160, and Gag).

55. In one embodiment, it is contemplated herein that the recombinant cells can comprise hsa-miR-431 and nucleic acids that encode any combination of the immunoglobulins, recombinant proteins, and recombinant peptides disclosed herein.

### 4. Delivery of the compositions to cells

56. It is understood and herein contemplated that the disclosed hsa-miR-431 and nucleic acid encoding an immunoglobulin gene are comprised within a recombinant cell. Cells appropriate for recombinant expression of immunoglobulins are known in the art and include but are not limited to Human Embryonic Kidney (HEK293) cells and Chinese Hamster Ovary (CHO) cells. Thus, in one aspect, disclosed herein are recombinant cells comprising hsa-miR-431 and at least one immunoglobulin encoding nucleic acid, wherein the recombinant cell is a HEK293 cell or a CHO cell. Also disclosed are recombinant cells comprising hsa-miR-431 and at least one immunoglobulin encoding nucleic acid, wherein the recombinant cell is a HEK293 cell or a CHO cell, wherein the immunoglobulin encoded by the nucleic acid comprises an immunoglobulin heavy chain gene, an immunoglobulin light chain gene, a single chain variable region (scFv), an agnathan variable lymphocyte receptor (VLR), diabody, bi-specific immunoglobulin gene, or an immunoglobulin fusion construct.

57. There are a number of compositions and methods which can be used to deliver nucleic acids to cells, either in vitro or in vivo. These methods and compositions can largely be broken down into two classes: viral based delivery systems and non-viral based delivery systems. For example, the nucleic acids can be delivered through a number of direct delivery systems such as, electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, include viral vectors, chemical transfectants, or physico-mechanical methods such as electroporation and direct diffusion of DNA. Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

### a) Nucleic acid based delivery systems

58. Transfer vectors can be any nucleotide construction used to deliver genes into cells (e.g., a plasmid), or as part of a general strategy to deliver genes, e.g., as part of recombinant retrovirus or adenovirus.

59. As used herein, plasmid or viral vectors are agents that transport the disclosed nucleic acids, the microRNA hsa-miR-431 and immunoglobulin encoding nucleic acids disclosed herein into the cell without degradation and include a promoter yielding expression of the gene in the cells into which it is delivered. Viral vectors are, for example, Adenovirus, Adeno-associated virus, Herpes virus, Lenti virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

60. Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promotor cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans. In these and other vector systems, the miRNA mimic expression construct can be designed and integrated into the vector so as to be expressed the miRNA in a variety of designs including but not limited to a pri-miRNA, a pre-miRNA, a short hairpin, a miRNA-like construct embedded in synthetic scaffold, or as a double stranded RNA.

### (1) Retroviral Vectors

61. A retrovirus is an animal virus belonging to the virus family of Retroviridae, including any types, subfamilies, genus, or tropisms (e.g., Lentivirus). Retroviral vectors, in general, are described by Verma, I.M., Retroviral vectors for gene transfer.

62. A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed, and upon replication be packaged into a new retroviral particle. This amount of nucleic acid is sufficient for the delivery of a one to many genes depending on the size of each transcript. It is preferable to include either positive or negative selectable markers along with other genes in the insert.

63. Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### (2) Adenoviral Vectors

64. The construction of replication-defective adenoviruses has been described. The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites. Recombinant adenoviruses achieve gene transduction by binding to specific cell surface receptors, after which the virus is internalized by receptor-mediated endocytosis, in the same manner as wild type or replication-defective adenovirus

65. A viral vector can be one based on an adenovirus which has had the E1 gene removed and these virons are generated in a cell line such as the CHO and HEK293 cell lines. In another preferred embodiment both the E1 and E3 genes are removed from the adenovirus genome.

### (3) Adeno-asscociated viral vectors

66. Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

67. In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus.

68. Typically the AAV and B19 coding regions have been deleted, resulting in a safe, noncytotoxic vector. The AAV ITRs, or modifications thereof, confer infectivity and site-specific integration, but not cytotoxicity, and the promoter directs cell-specific expression. d United states Patent No. 6,261,834 is herein reference for material related to the AAV vector.

69. The disclosed vectors thus provide DNA molecules which are capable of integration into a mammalian chromosome without substantial toxicity.

70. The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### (4) Large payload viral vectors

71. Molecular genetic experiments with large human herpesviruses have provided a means whereby large heterologous DNA fragments can be cloned, propagated and established in cells permissive for infection with herpesviruses. These large DNA viruses (herpes simplex virus (HSV) and Epstein-Barr virus (EBV), have the potential to deliver fragments of human heterologous DNA > 150 kb to specific cells. EBV recombinants can maintain large pieces of DNA in the infected B-cells as episomal DNA. Individual clones carried human genomic inserts up to 330 kb appeared genetically stable The maintenance of these episomes requires a specific EBV nuclear protein, EBNA1, constitutively expressed during infection with EBV. Additionally, these vectors can be used for transfection, where large amounts of protein can be generated transiently in vitro. Herpesvirus amplicon systems are also being used to package pieces of DNA > 220 kb and to infect cells that can stably maintain DNA as episomes.

72. Other useful systems include, for example, replicating and host-restricted non-replicating vaccinia virus vectors.

### b) Non-nucleic acid based systems

73. The disclosed compositions can be delivered to the target cells in a variety of ways. For example, the compositions can be delivered through electroporation, or through lipofection, or through calcium phosphate precipitation. The delivery mechanism chosen will depend in part on the type of cell targeted and whether the delivery is occurring for example in vivo or in vitro.

74. Thus, the compositions can comprise, in addition to the disclosed microRNA hsa-miR-431 and immunoglobulin encoding nucleic acids or vectors for example, lipids such as liposomes, such as cationic liposomes (e.g., DOTMA, DOPE, DC-cholesterol) or anionic liposomes. Liposomes can further comprise proteins to facilitate targeting a particular cell, if desired. Administration of a composition comprising a compound and a cationic liposome can be administered to the blood afferent to a target organ or inhaled into the respiratory tract to target cells of the respiratory tract. Furthermore, the compound can be administered as a component of a microcapsule that can be targeted to specific cell types, such as macrophages, or where the diffusion of the compound or delivery of the compound from the microcapsule is designed for a specific rate or dosage.

75. In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTlN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the disclosed nucleic acid or vector can be delivered *in vivo* by electroporation, the technology for which is available from Genetronics, Inc. (San Diego, CA) as well as by means of a SONOPORATION machine (ImaRx Pharmaceutical Corp., Tucson, AZ).

76. The materials may be in solution, suspension (for example, incorporated into microparticles, liposomes, or cells). These may be targeted to a particular cell type via antibodies, receptors, or receptor ligands. These techniques can be used for a variety of other specific cell types. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration.

77. Nucleic acids that are delivered to cells which are to be integrated into the host cell genome, typically contain integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can become integrated into the host genome.

78. Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

### 5. Expression systems

81. The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### a) Viral Promoters and Enhancers

82. Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication. The immediate early promoter of the human cytomegalovirus is conveniently obtained as a *Hind*III E restriction fragment. Of course, promoters from the host cell or related species also are useful herein.

83. Enhancer generally refers to a sequence of DNA that functions at no fixed distance from the transcription start site and can be either 5' or 3' to the transcription unit. Furthermore, enhancers can be within an intron as well as within the coding sequence itself. They are usually between 10 and 300 bp in length, and they function in cis. Enhancers f unction to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, -fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

84. In certain embodiments the promoter and/or enhancer region can act as a constitutive promoter and/or enhancer to maximize expression of the region of the transcription unit to be transcribed. Thus, in one embodiment disclosed herein are recombinant cells comprising hsa-miR-431 and at least one immunoglobulin encoding nucleic acid wherein the expression of hsa-miR-431 is constitutive. In such circumstances, hsa-miR-431 can be operationally linked to the constitutive promoter. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR.

85. In other embodiments, the promoter and/or enhancer region can act as an inducible promoter and/or enhancer to regulate expression of the region of the transcript to be transcribed. The promoter and/or enhancer may be specifically activated either by light, temperature, or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs. Other examples of inducible promoter systems include but are not limited to GAL4 promoter, Lac promoter, Cre recombinase (such as in a cre-lox inducible system), metal-regulated systems such as metallothionein, Flp-*FRT* recombinase, alcohol dehydrogenase I (alcA) promoter, and steroid regulated systems, such as, estrogen receptor (ER) and glucocorticoid receptor (GR). Inducible systems can also comprise inducible stem loop expression systems. Thus, in one embodiment disclosed herein are recombinant cells comprising hsa-miR-431 and at least one immunoglobulin encoding nucleic acid wherein the expression of hsa-miR-431 is inducible.

86. It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

87. Expression vectors used in eukaryotic host cells (yeast, fungi, insect, plant, animal, human or nucleated cells) may also contain sequences necessary for the termination of transcription which may affect mRNA expression. These regions are transcribed as polyadenylated segments in the untranslated portion of the mRNA encoding tissue factor protein. The 3' untranslated regions also include transcription termination sites. It is preferred that the transcription unit also contains a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### b) Markers

88. The viral vectors can include nucleic acid sequence encoding a marker product. This marker product is used to determine if the gene has been delivered to the cell and once delivered is being expressed. Preferred marker genes are the *E. Coli* lacZ gene, which encodes β-galactosidase, and green fluorescent protein.

89. In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR- cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

90. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, mycophenolic acid, or hygromycin,. The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### 6. Sequence similarities

91. It is understood that as discussed herein the use of the terms homology and identity mean the same thing as similarity. Thus, for example, if the use of the word homology is used between two non-natural sequences it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

92. In general, it is understood that one way to define any known variants and derivatives or those that might arise, of the disclosed genes and proteins herein, is through defining the variants and derivatives in terms of homology to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to the stated sequence or the native sequence. Those of skill in the art readily understand how to determine the homology of two proteins or nucleic acids, such as genes. For example, the homology can be calculated after aligning the two sequences so that the homology is at its highest level.

93. Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

94. It is understood that any of the methods typically can be used and that in certain instances the results of these various methods may differ, but the skilled artisan understands if identity is found with at least one of these methods, the sequences would be said to have the stated identity, and be disclosed herein.

95. For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### 7. Hybridization/selective hybridization

96. The term hybridization typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

97. Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

98. Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.

99. Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.

100. Just as with homology, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

### 8. Transgenic animals.

101. It is understood and herein contemplated that in one aspect, the recombinant cells disclosed herein can be present in a non-human transgenic animal. Thus, in one aspect, disclosed herein are non-human transgenic animals comprising any recombinant cell disclosed herein. For example, disclosed herein are non-human transgenic animals comprising exogenous hsa-miR-431 Also disclosed are non-human transgenic animals comprising exogenous hsa-miR-431 and at least one immunoglobulin encoding nucleic acid. Also disclosed are non-human transgenic animals comprising exogenous hsa-miR-431 and at least one immunoglobulin encoding nucleic acid, wherein the immunoglobulin encoding nucleic acid encodes an immunoglobulin heavy chain gene, an immunoglobulin light chain gene, a single chain variable region (scFv), an agnathan variable lymphocyte receptor (VLR), diabody, bi-specific immunoglobulin gene, or an immunoglobulin fusion construct.

102. The non-human transgenic animals of this invention can be made by methods known in the art. For the purposes of generating a non-human transgenic animal, screening the non-human transgenic animal for the presence of a transgene and other methodology regarding transgenic animals, please see U.S. Pat. No. 6,111,166. For example, the non-human transgenic animals of this invention can be made by a) injecting a transgene comprising a nucleic acid encoding hsa-miR-431 functionally linked to an expression sequence and/or a transgene comprising a nucleic acid encoding an immunoglobulin functionally linked to an expression sequence into a non-human embryo and b) allowing the embryo to develop into an animal. This can further comprise crossing the animal with a second animal to produce a third animal. Specifically contemplated are transgenic non-human mammals such as a transgenic mouse, rat, sheep, dog, cat, cow, horse, rabbit, and pig.

103. Expression of the transgene can be controlled by any of the inducible or constitutive expression systems disclosed herein.

104. It is understood that those of skill in the art understand that if a composition or method meets any one of these criteria for determining hybridization either collectively or singly it is a composition or method that is disclosed herein.

### C. Methods of Enhancing Recombinant Antibody production

105. It is understood and herein contemplated that one of the uses of the recombinant cells disclosed herein is to increase recombinant proteins, peptides, and/or antibody production and purification in high throughput protein, peptide, and/or antibody production methods. Thus, in one aspect disclosed herein are methods of enhancing recombinant protein, peptide, and/or antibody production from a cell comprising artificially introducing or recombinantly expressing hsa-miR-431 in a cell comprising at least one nucleic acid encoding an immunoglobulin, exogenous protein, or exogenous peptide. For example , disclosed herein are methods of enhancing recombinant antibody production from a cell comprising recombinantly expressing hsa-miR-431 in a cell comprising at least one immunoglobulin encoding nucleic acid Also disclosed are methods of enhancing recombinant antibody production from a cell comprising recombinantly expressing hsa-miR-431 in a cell comprising at least one immunoglobulin encoding nucleic acid wherein the immunoglobulin encoding nucleic acid encodes an immunoglobulin heavy chain gene, an immunoglobulin light chain gene, a single chain variable region (scFv), an agnathan variable lymphocyte receptor (VLR), diabody, bi-specific immunoglobulin gene, or an immunoglobulin fusion construct.

106. In another aspect, disclosed herein are methods of enhancing recombinant protein production from a cell comprising recombinantly expressing hsa-miR-431 in a cell comprising at least one nucleic acid encoding an exogenous protein or peptide Also disclosed are methods of enhancing recombinant protein production from a cell comprising recombinantly expressing hsa-miR-431 mimic in a cell comprising at least one nucleic acid encoding an exogenous protein or peptide wherein the exogenous protein is selected from the group consisting of Tissue Plasminogen Activator (TPA), Erythropoietin (EPO), Granulocyte colony-stimulating factor (G-CSF), Interferon, 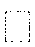 -galactosidase A, 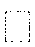 -L-iduronidase, N-acetylgalactosamine-4-sulfatase, Glucocerebrosidase, Dornase alfa, Hepatitis B virus Envelope (Env) Protein, Factor VII, recombinant human growth hormone (rHGH), biosynthetic human insulin (BSI), follicle-stimulating hormone (FSH), recombinant cholera toxin B, diphtheria toxoid, tetanus toxoid, pertussis toxoid, Hepatitis B surface antigen (HBsAg), HPV capsid protein, and recombinant Human Immunodeficiency Virus (HIV) proteins (Pol, gp41, gp120, gp160, and Gag).

107. It is understood that the disclosed methods work with any recombinant cell system including but not limited to CHO and HEK293 cells.

### D. Examples

108. The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### 1. Example 1

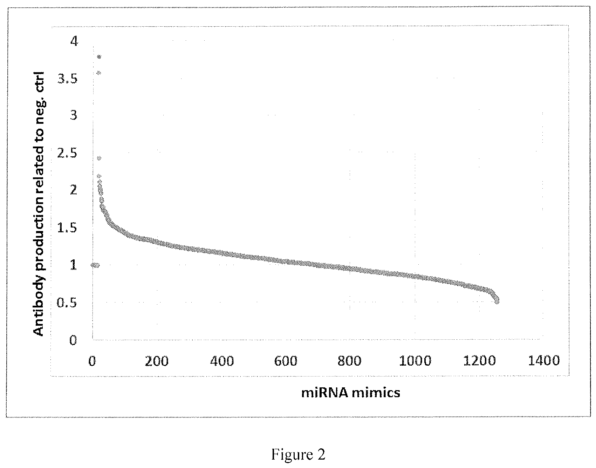

109. In terms of therapeutic proteins at industrial scales, the most successfully used mammalian cell line for antibody production is the Chinese Hamster Ovary (CHO) cell line, due to its unique characters for industrial application: easy culture in in suspension, easy adaptation to a modified media conditions, and high quality of endpoint therapeutic antibodies. Biopharmaceutical companies are of increasingly interest in exploring innovative strategies that can improve the antibody production. However, only till recent years, the most significant progress in this field is from CHO cell culture modification and feeding strategies that resulted in antibody production 2-6g /per liter or so. Although most recent report showed that single gene engineering may improve product quality, the productivity of antibody is still not comparable to traditional medium optimization. The unsuccessful single gene modification to significantly enhance antibody productivity has led to a paradigm shift to on how to the control of multiple pathways. MicroRNAs have been demonstrated to regulate multiple pathways that are involved in diverse cellular activities which have been suggested as therapeutic targets for cell engineering. Herein miRNA mimics were screened in CHO cells expressing human antibody (Figure 2). Seven miRNA mimics when introduced into CHO cells were found to increase antibody production more than 2∼4 fold, as compared to mock control (Table 1). The miRNA sequences and accession numbers are shown in Table 2.

### a) Transfection & Screen protocol

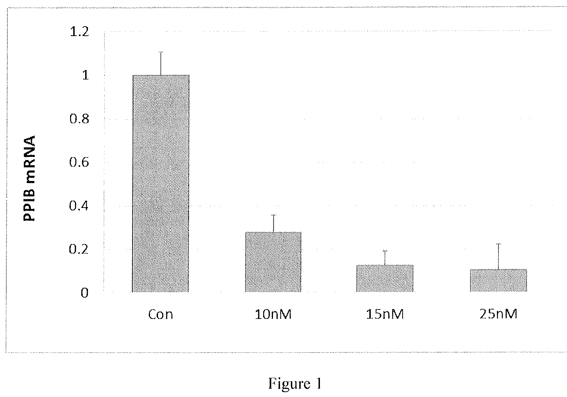

Highly efficient delivery of miRNA into CHO cells was optimized in the lab (Figure 1). For HTS miRNA transfection in the primary screen, miRIDIAN microRNA Mimics (Thermo Fisher) were reverse transfected into CHO cells at a final concentration of miRNA at 20nM in the presence of 0.5% DF1, with 15000 cells/per 96 well. DF1 was diluted in serum-free medium (OPTI-MEM, Invitrogen Inc.) for 5 minutes prior to adding to 96-well culture plates with pre-added 5ul of 200nM miRNAs. The DF1 and miRNA mixture was then incubated for 20 minutes at room temperature prior to addition of cells in serum-free medium CHO medium (GIBCO). Transfected cells were then cultured for 7 days at 37°C, 5% CO₂. Afterwards, the media was removed for an ELISA assay. For these studies, mouse anti human IgG antibody (ab7500) was diluted in 1x PBS buffer and incubated in 96-well plates overnight in the cold room. After washing with 3X with KPL wash buffer, 50ul of each supernatant derived from miRNA mimic transfected CHO cells was added to the plate, and incubated at room temperature for 2 hours. Following an additional wash step, HRP-conjugated antibody goat anti human IgG (ab87422) was added to develop ELISA. The assay plates were then evaluated on a spectrophotometer at wavelength at 450nm. The values were then normalized to non-targeting control (with NTC = 1.0).

### b) miRNA Mimic Reagents

110. The Human miRIDIAN miRNA Mimic Library (19.0) obtained from Thermo Fisher Scientific (now GE Dharmacon) was used for the primary miRNA screen. For the miRNA primary screen, the total miRNA concentration during transfection was 20nM.

### c) Data normalization and data analysis

111. Mock controls and non-targeting controls were included in each plate and the signal from each hit was compared to each of these controls. Data analysis was performed using standard techniques recognized by the art.

**Table 1. Top hits identified from the miRNA mimics screen. 7 miRNA mimics were shown to increase antibody production, of these, two miRNAs were shown to increase antibody production from 3.5-3.8 x fold assuming a linear response in the ELISA.**

| **Neg** | **1** |
|---|---|
| **hsa-miR-431** | **3.795455** |
| **hsa-miR-623** | **3.572538** |
| **hsa-miR-4325** | **2.434628** |
| **hsa-miR-3129** | **2.18906** |
| **hsa-miR-3127** | **2.106154** |
| **hsa-miR-873** | **2.049802** |
| **hsa-miR-612** | **2.019685** |

**Table 2. Sequences and accession numbers of the identified microRNAs.**

| Name | Accession | Sequence |
|---|---|---|
| hsa-miR-431 | MI0001721 | UGUCUUGCAGGCCGUCAUGCA |
| hsa-miR-623 | MI0003637 | AUCCCUUGCAGGGGCUGUUGGGU |
| hsa-miR-4325 | MI0015865 | UUGCACUUGUCUCAGUGA |
| hsa-miR-3129 | MI0014146 | GCAGUAGUGUAGAGAUUGGUUU |
| hsa-miR-3127 | MI0014144 | AUCAGGGCUUGUGGAAUGGGAAG |
| hsa-miR-873 | MI0005564 | GCAGGAACUUGUGAGUCUCCU |
| hsa-miR-612 | MI0003625 | GCUGGGCAGGGCUUCUGAGCUCCUU |

## Claims

1. A recombinant cell comprising hsa-miR-431 and at least one nucleic acid encoding an exogenous protein or immunoglobulin.

2. The recombinant cell of claim 1, wherein the expression of hsa-miR-431 is constitutive.

3. The recombinant cell of claim 1, wherein the expression of hsa-miR-431 is inducible.

4. The recombinant cell of claim 1, wherein the recombinant cell is a CHO or HEK293 cell.

5. The recombinant cell of claim 1, wherein the at least one nucleic acid encodes an immunoglobulin heavy chain gene, an immunoglobulin light chain gene, a single chain variable region (scFv), an agnathan variable lymphocyte receptor (VLR), diabody, bi-specific immunoglobulin gene, or an immunoglobulin fusion construct.

6. The recombinant cell of claim 1, wherein the at least one nucleic acid encodes a recombinant protein selected from the group consisting of Tissue plasminogen activator (TPA), Erythropoietin (EPO), Granulocyte colony-stimulating factor (G-CSF), Interferon, oc-galactosidase A, a-L-iduronidase, N-acetylgalactosamine-4-sulfatase, Glucocerebrosidase, Dornase alfa, Hepatitis B virus Envelope (Env) Protein, Factor VII, recombinant human growth hormone (rHGI 1), biosynthetic human insulin (BSI), follicle-stimulating hormone (FSH), recombinant cholera toxin B, diphtheria toxoid, tetanus toxoid, pertussis toxoid, Hepatitis B surface antigen (HBsAg), HPV capsid protein, and recombinant Human Immunodeficiency Virus (HIV) proteins (Pol, gp41, gp I 20, gp I 60, and Gag).

7. A non-human transgenic animal comprising the recombinant cell of claim 1.

8. A non-human transgenic animal, comprising hsa-miR-431.

9. An *in vitro* method of enhancing recombinant antibody production from a cell comprising recombinantly expressing hsa-miR-431 in a cell comprising at least one immunoglobulin gene.

## Patentansprüche

1. Eine rekombinante Zelle, die hsa-miR-431 und mindestens eine für ein exogenes Protein oder Immunglobulin kodierende Nukleinsäure umfasst.

2. Die rekombinante Zelle nach Anspruch 1, wobei die Expression von hsa-miR-431 konstitutiv ist.

3. Die rekombinante Zelle nach Anspruch 1, wobei die Expression von hsa-miR-431 induzierbar ist.

4. Die rekombinante Zelle nach Anspruch 1, wobei die rekombinante Zelle eine CHO- oder HEK293-Zelle ist.

5. Die rekombinante Zelle nach Anspruch 1, wobei die mindestens eine Nukleinsäure für ein schweres Ketten-Gen eines Immunglobulins, ein leichtes Ketten-Gen eines Immunglobulins, eine variable Region einer Einzelkette (scFv), einen agnathanen variablen Lymphozytenrezeptor (VLR), einen Diabody, ein Gen eines bi-spezifischen Immunglobulins, oder ein Immunglobulin-Fusionskonstrukt kodiert.

6. Die rekombinante Zelle nach Anspruch 1, wobei die mindestens eine Nukleinsäure für ein rekombinantes Protein kodiert, ausgewählt aus der Gruppe bestehend aus gewebespezifischem Plasminogenaktivator (TPA), Erythropoietin (EPO), Granulozyten-Kolonie-stimulierender Faktor (G-CSF), Interferon, Oc-Galactosidase A, α-L-Iduronidase, N-Acetylgalactosamin-4-Sulfatase, Glucocerebrosidase, Dornase alfa, Hepatitis-B-Virus Hüllprotein (Env), Faktor VII, rekombinantes humanes Wachstumshormon (rHGI 1), biosynthetisches Humaninsulin (BSI), follikelstimulierendes Hormon (FSH), rekombinantes Choleratoxin B, Diphtherietoxoid, Tetanustoxoid, Pertussistoxoid, Hepatitis-B-Oberflächenantigen (HBsAg), HPV-Kapsidprotein, und rekombinante Humanes-Immundefizienz-Virus (HIV) -Proteine (Pol, gp41, gp I 20, gp I 60, and Gag).

7. Ein nicht-humanes transgenes Tier, das die rekombinante Zelle nach Anspruch 1 umfasst.

8. Ein nicht-humanes transgenes Tier, das hsa-miR-431 umfasst.

9. Ein *in vitro* Verfahren zur Steigerung der rekombinanten Antikörperproduktion einer Zelle, umfassend ein rekombinantes Exprimieren von hsa-miR-431 in einer Zelle, die mindestens ein Immunglobulin-Gen umfasst.

## Revendications

1. Cellule recombinante comprenant hsa-miR-431 et au moins un acide nucléique codant pour une protéine ou immunoglobuline exogène.

2. Cellule recombinante selon la revendication 1, dans laquelle l'expression de hsa-miR-431 est constitutive.

3. Cellule recombinante selon la revendication 1, dans laquelle l'expression de hsa-miR-431 est inductible.

4. Cellule recombinante selon la revendication 1, dans laquelle la cellule recombinante est une cellule CHO ou HEK293.

5. Cellule recombinante selon la revendication 1, dans laquelle l'au moins un acide nucléique code pour un gène de chaîne lourde d'immunoglobuline, un gène de chaîne légère d'immunoglobuline, une région variable simple chaîne (scFv), un récepteur lymphocytaire variable d'agnathe (VLR), un diabody, un gène d'immunoglobuline bispécifique, ou une construction de fusion d'immunoglobuline.

6. Cellule recombinante selon la revendication 1, dans laquelle l'au moins un acide nucléique code pour une protéine recombinante sélectionnée dans le groupe consistant en un activateur tissulaire du plasminogène (TPA), une érythropoïétine (EPO), un facteur de stimulation des colonies de granulocytes (G-CSF), un interféron, une oc-galactosidase A, une a-L-iduronidase, une N-acétylgalactosamine-4-sulfatase, une glucocérébrosidase, une dornase alfa, une protéine d'enveloppe du virus de l'hépatite B (Env), un facteur VII, une hormone de croissance humaine recombinante (rHGI 1), une insuline humaine biosynthétique (BSI), une folliculo-stimuline (FSH), une toxine cholérique B recombinante, une anatoxine diphtérique, une anatoxine tétanique, une anatoxine pertussique, un antigène de surface de l'hépatite B (HBsAg), une protéine de capside de HPV, et des protéines recombinantes du virus de l'immunodéficience humaine (VIH) (Pol, gp41, gpl20, gpl60 et Gag).

7. Animal transgénique non humain comprenant la cellule recombinante selon la revendication 1.

8. Animal transgénique non humain, comprenant hsa-miR-431.

9. Procédé *in vitro* d'amélioration de la production d'un anticorps recombinant à partir d'une cellule comprenant l'expression recombinante de hsa-miR-431 dans une cellule comprenant au moins un gène d'immunoglobuline.
